# EUROPÄISCHE PATENTSCHRIFT

(11) **EP 4 096 593 B1**
(45) Veröffentlichungstag und Bekanntmachung des Hinweises auf die Patenterteilung: **31.01.2024**
(21) Anmeldenummer: 20824428.5
(22) Anmeldetag: 01.12.2020
(51) Int. Cl.: A61F 5/058, B65B 31/02, B65B 43/36, B65B 31/04

(54) **VERFAHREN ZUM BEFÜLLEN EINES FLEXIBLEN ELEMENTES ZUR STÜTZUNG UND STABILISIERUNG EINES VERLETZTEN**
METHOD FOR FILLING A FLEXIBLE ELEMENT FOR SUPPORTING AND STABILISING AN INJURED PERSON
PROCÉDÉ DE REMPLISSAGE D'UN ÉLÉMENT SOUPLE POUR SUPPORTER ET STABILISER UNE PERSONNE BLESSÉE

(30) Priorität: 28.01.2020 AT 500642020; 10.03.2020 AT 501942020
(43) Veröffentlichungstag der Anmeldung: 07.12.2022
(73) Patentinhaber: Kohlbrat & Bunz Gesellschaft m.b.H, 5550 Radstadt (AT)
(72) Erfinder: RUGFELT, Haakan, 23941 Falsterbo (SE)
(74) Vertreter: Torggler & Hofmann Patentanwälte - Innsbruck
(86) Internationale Anmeldenummer: PCT/AT2020/060428
(87) Internationale Veröffentlichungsnummer: WO 2021/151128

(56) Entgegenhaltungen:
- EP-A1- 2 130 769
- WO-A1-2013/044277
- JP-A- H11 236 012

## Beschreibung

Die Erfindung betrifft ein Verfahren zur Befüllung eines flexiblen Elementes, für die Stützung und Stabilisierung eines Verletzten, mit einem losen Granulat, wobei das Element zwei an Stellen im Innenraum und unter Belassung einer Füllöffnung entlang des Randes miteinander verbundene Materialbahnen aufweist. Auch als Vakuummatratzen und Vakuumschienen bezeichnete Stabilisierungseinrichtungen für Transport und Immobilisierung von Verletzten weisen eine Hülle aus einer luftdichten Kunststofffolie und eine gleichmäßige Füllung aus einem Kunststoffgranulat, insbesondere aus geschäumten Polystyrolkugeln, auf und können nach der Anpassung und Fixierung an einem ruhig zu stellenden Körperteil mittels einer Saugpumpe evakuiert werden. Dies führt zu einer dichten Packung des lose eingefüllten Granulats und somit zu einer Versteifung des flexiblen Elements, das auf diese Weise eine im Wesentlichen starre Manschette bildet. Für eine möglichst universelle Fixierung am Körperteil sind Befestigungsgurte in Umfangsrichtung des Körperteils angeordnet und mit üblichen einstellbaren Verbindungsbeschlägen versehen. Da eine gleichmäßige Verteilung des Granulates erzielt werden muss, sind Vakuummatratzen und -schienen hauptsächlich für eine horizontale Einsatzlage geeignet. Bei Verwendung in einer nicht horizontalen Lage, beispielsweise bei sitzenden Verletzten, besteht die Gefahr, dass die Füllung in einem höheren Bereich eine dünnere Schicht bildet oder sogar fehlt, weil sie sich während der Handhabung von oben nach unten verlagert hat.

Aus der WO 2013/044277 ist ein Verfahren zur Herstellung einer derartigen Stabilisierungseinrichtung bekannt, in dem die beiden Folien an drei Seiten entlang des Randes und in einem Vierpunktraster im Inneren so miteinander verbunden werden, dass Kanäle parallel zu mindestens einem Seitenrand verbleiben. Bei der Befüllung mit Granulat wird in jeden Kanal eine Fülllanze eingeschoben und durch die Fülllanzen Granulat zugeführt, wobei die Fülllanzen entsprechend dem Füllfortschritt wieder herausgezogen werden. Schließlich werden dann die Folien auch am offenen oberen Querrand miteinander verbunden. Die inneren Verbindungen sind dabei vorgesehen, um eine annähernd gleichmäßige Distanzierung der beiden Folien über die gesamte Fläche und damit eine annähernd gleiche Schichtdicke des Granulates zu gewährleisten.

Die Erfindung hat es sich zur Aufgabe gestellt, das Füllverfahren zu vereinfachen, wobei die inneren Verbindungen auch unabhängig von einer bestimmten Form und einem bestimmten Raster hergestellt werden können.

Dies wird erfindungsgemäß dadurch erreicht, dass ein Luftdruckunterschied zwischen dem Innenraum und der äußeren Umgebung erzeugt wird, der den Innenraum vor der Befüllung mit dem Granulat aufweitet.

Prinzipiell gibt es dabei die Möglichkeit, den Luftdruck außen zu verringern, innen zu erhöhen, oder beide Maßnahmen gleichzeitig anzuwenden. Bevorzugt ist vorgesehen, dass das Element in eine Unterdruckkammer eingesetzt wird, wobei die Füllöffnung von außen zugänglich ist, und in der Unterdruckkammer der Unterdruck erzeugt wird, der den Innenraum aufweitet.

In einer bevorzugten Ausführung ist dabei vorgesehen, dass die Füllöffnung in einem aus Bereichen der beiden Materialbahnen gebildeten Füllstutzen vorgesehen wird, der nach Einbringung des Granulates geschlossen wird. Dabei ist weiters bevorzugt vorgesehen, dass das Element in der Unterdruckkammer am Füllstutzen aufgehängt wird, wobei dessen Öffnung nach außen ragt.

Das Befüllen mit dem Granulat erfolgt bevorzugt mittels Schwerkraft nach bzw. gleichzeitig mit der Aufweitung des Innenraumes durch Absaugen der Umgebungsluft. Wird in den Innenraum Luft eingeblasen, kann das Granulat aber auch der Luft bereits beigegeben werden.

Das flexible Element kann aus zwei luftdichten Folien gebildet sein, von denen zumindest eine ein Ventil aufweist, um es nach Anpassung an den Verletzten zu evakuieren. Es ist aber auch möglich, Materialbahnen aus einem luftdurchlässigen Vlies oder dergleichen zu verwenden, die nach der Befüllung zwischen zwei luftdichte Folien eingeschlossen werden, von denen zumindest eine das oben erwähnte Ventil aufweist. Somit kann dann die das befüllte Element enthaltende Hülle nach Anpassung an den Verletzten evakuiert werden.

Die Füllung besteht vor allem aus einem Granulat aus geschäumtem Polystyrol mit einem Gewicht zwischen 20 kg/m³ und 70 kg/m³, insbesondere von 60 kg/m³, wobei der mittlere Durchmesser der einzelnen Partikel zwischen 0,4 mm und 5 mm, insbesondere zwischen 0,5 mm und 2 mm ist. Die Maschenweite bzw. Porengröße der luftdurchlässigen Materialbahn ist selbstverständlich kleiner als der Durchmesser der Partikel. Dadurch ist auch der notwendige Luftwiderstand zur Erreichung des Druckunterschiedes zwischen dem Innenraum und der Umgebung gesichert.

Nachstehend wird nun die Erfindung anhand der Figuren der beiliegenden Zeichnung näher beschrieben, ohne darauf beschränkt zu sein. Es zeigen:
- Fig. 1: eine schematische Ansicht eines rechteckigen Elementes,
- Fig. 2: eine schematische Darstellung der Füllung des Elementes mit Granulat, und
- Fig. 3: eine schematische Draufsicht auf das Element während des Füllvorganges.

Ein Element 1, das je nach Größe und Formgebung zur Immobilisierung und Stabilisierung eines verletzten Körperteils, beispielsweise eines gebrochenen Armes oder Beines, oder einer verletzten Person geeignet ist, weist zwei Materialbahnen 2 aus einem luftdichten Kunststoff, beispielsweise aus einem Polyurethan, oder einem luftdurchlässigen Vlies auf, die entlang des Randes umlaufend und an geeigneten Stellen 3 im Inneren miteinander verbunden, insbesondere verschweißt sind. Der Innenraum 4 enthält ein Granulat 9 aus einem leichten Kunststoff, insbesondere aus einem geschäumten Polystyrol, wobei der Durchmesser der einzelnen Kugeln insbesondere zwischen 0,5 und 2 mm liegt. Eine der beiden Folien ist mit einem nicht gezeigten Ventil versehen, über das die im Inneren eingeschlossene Luft abgesaugt werden kann, wodurch das lose eingefüllte und durch äußere Einwirkung verschiebbare bzw. verlagerbare Granulat 9 in eine dichte Packung überführt wird, die das Element 1 ausreichend versteift, um eine Bewegung des verletzten Körpers bzw. Körperteils zu unterbinden.

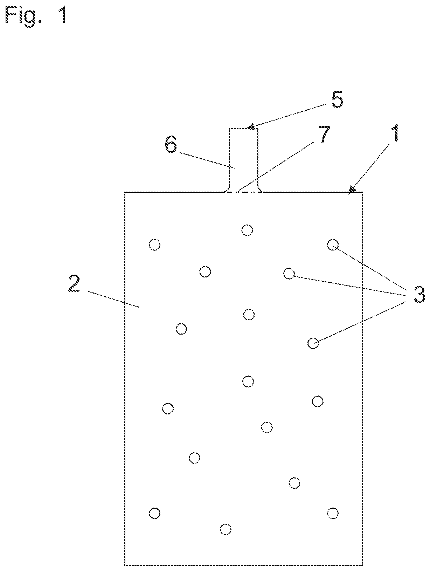

Wie in Fig. 1 gezeigt, wird ein derartiges Element 1 wie folgt hergestellt: zuerst werden zwei Materialbahnen 2 zugeschnitten, die jeweils einen Fortsatz aufweisen. Die beiden Materialbahnen 2 werden übereinander gelegt und an mehreren, voneinander beabstandeten Verbindungsstellen 3 direkt oder mittels Zwischenstücken im Inneren und entlang des Randes unter Belassung einer Füllöffnung 5 miteinander verschweißt. Die beiden Fortsätze ergänzen einander zu einem die Füllöffnung 5 aufweisenden Füllstutzen 6, sodass ein offenes sackähnliches Gebilde entsteht. Die Anordnung der Verbindungsstellen 3 unterliegt keinem bestimmten Schema, und kann nach praktischen Gegebenheiten, abhängig vom Verwendungszweck gewählt werden.

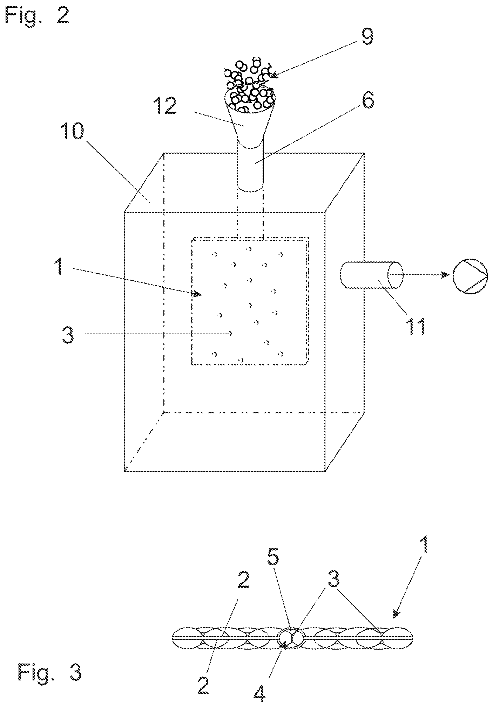

Fig. 2 zeigt schematisch eine Unterdruckkammer 10, deren Innenraum über einen Absaugstutzen 11 mit einem Unterdruckerzeuger verbunden ist. Die Unterdrucckammer 10 weist eine obere Öffnung auf. Das Element 1 wird nun in die Unterdruckkammer 10 eingebracht, wobei der durch die Fortsätze der Materialbahnen 2 gebildete Füllstutzen 6 durch die obere Öffnung gedichtet nach außen geführt wird. Die Füllöffnung 5 liegt daher außerhalb der Unterdruckkammer 10 und ist unter Normaldruck. Oberhalb des Füllstutzens 6 ist ein schematisch gezeigter Fülltrichter 12 vorgesehen, über den Granulat 9 insbesondere durch Schwerkraft zugeführt werden kann. Nun wird in der Unterdruckkammer 10 ein Unterdruck erzeugt, durch den die beiden Materialbahnen 2 auseinander gezogen werden, sodass sich der unter Normaldruck stehende Innenraum 4 vergrößert, wie schematisch in Fig. 3 dargestellt ist, und Granulat 9 in den Innenraum 4 einfließt bis die benötigte Menge erreicht ist. Die Füllung verteilt sich dabei ohne Schwierigkeiten zwischen den Verbindungsstellen 3.

Anschließend wird das gefüllte Element 1 aus der Unterdruckkammer 10 entnommen, und die Füllöffnung 5 verschlossen, insbesondere durch eine Naht zwischen den beiden Materialbahnen entlang der in Fig. 1 strichpunktiert gezeigten Linie 7. Die beiden nicht mehr benötigten Fortsätze der Materialbahnen 2 werden dann vorzugsweise abgeschnitten. Das Element 1 ist einsatzbereit, wenn als Materialbahnen luftdicht Kunststofffolien verwendet werden. Bestehen die Materialbahnen aus einem luftdurchlässigen Kunststoffvlies oder dergleichen, so wird das befüllte Element abschließend in eine Hülle aus zwei luftdichten Kunststofffolien eingeschlossen, die dann zur Versteifung des Elementes 1 wiederum evakuiert werden kann.

## Patentansprüche

1. Verfahren zum Befüllen eines flexiblen Elementes (1) zur Stützung und Stabilisierung eines Verletzten mit einem losen Granulat (9), wobei das Element (1) zwei - an Stellen (3) im Innenraum (4) und, unter Belassung einer Füllöffnung (5), entlang des Randes - miteinander verbundene Materialbahnen (2) aufweist, **dadurch gekennzeichnet, dass** ein Luftdruckunterschied zwischen dem Innenraum (4) und der äußeren Umgebung erzeugt wird, der den Innenraum (4) vor der Befüllung mit dem Granulat (9) aufweitet.

2. Verfahren nach Anspruch 1, **dadurch gekennzeichnet, dass** der Luftdruckunterschied zwischen dem Innenraum (4) und der äußeren Umgebung durch Absaugen von Umgebungsluft hergestellt wird.

3. Verfahren nach Anspruch 2, **dadurch gekennzeichnet, dass** das Element (1) in eine Unterdruckkammer (10) eingesetzt wird, wobei die Füllöffnung (5) von außen zugänglich ist, und in der Unterdruckkammer (10) der Unterdruck erzeugt wird, der den Innenraum (4) aufweitet.

4. Verfahren nach Anspruch 1, **dadurch gekennzeichnet, dass** der Luftdruckunterschied zwischen dem Innenraum (4) und der äußeren Umgebung durch Einblasen von Luft durch die Füllöffnung (5) hergestellt wird.

5. Verfahren nach Anspruch 4, **dadurch gekennzeichnet, dass** das lose Granulat (9) dem durch die Füllöffnung (5) eingebrachten Luftstrom beigegeben wird.

6. Verfahren nach Anspruch 2 oder 4, **dadurch gekennzeichnet, dass** das lose Granulat (9) nach der Aufweitung des Innenraumes (4) durch die Füllöffnung (5) mittels Schwerkraft eingebracht wird.

7. Verfahren nach einem der Ansprüche 1 bis 6, **dadurch gekennzeichnet, dass** die Füllöffnung (5) in einem aus Bereichen der beiden Materialbahnen (2) gebildeten Füllstutzen (6) vorgesehen wird, der nach Einbringung des Granulats (9) geschlossen wird.

8. Verfahren nach Anspruch 3 und 7, **dadurch gekennzeichnet, dass** das Element (1) in der Unterdruckkammer (10) am Füllstutzen (6) aufgehängt wird.

9. Verfahren nach einem der Ansprüche 1 bis 8, **dadurch gekennzeichnet, dass** das Granulat (9) aus geschäumtem Polystyrol mit einem Gewicht zwischen
20 kg/m³ und 70 kg/m³ besteht.

10. Verfahren nach einem der Ansprüche 1 bis 8, **dadurch gekennzeichnet, dass** das Granulat (9) aus geschäumtem Polystyrol mit einem Durchmesser zwischen 0,4 mm und 5 mm besteht.

11. Verfahren nach Anspruch 9 oder 10, **dadurch gekennzeichnet, dass** das Granulat (9) aus geschäumtem Polystyrol mit einem Gewicht von 60 kg/m³ und einem Durchmesser zwischen 0,5 mm und 2 mm besteht.

12. Verfahren zum Herstellen eines flexiblen Elementes (1) zur Stützung und Stabilisierung eines Verletzten, bei dem ein Verfahren nach einem der Ansprüche 1 bis 11 zum Befüllen des flexiblen Elementes (1) angewandt wird.

13. Verfahren nach Anspruch 12, **dadurch gekennzeichnet, dass** die beiden Materialbahnen (2) aus luftdichten Folien gebildet sind, von denen zumindest eine ein Ventil aufweist, sodass das mit dem Granulat (9) befüllte Element (1) nach Anpassung an den Verletzten evakuierbar ist.

14. Verfahren nach Anspruch 12, **dadurch gekennzeichnet, dass** die beiden Materialbahnen (2) aus luftdurchlässigem Vlies gebildet sind, und nach Befüllung mit dem Granulat (9) in eine Hülle aus luftdichten Folien eingeschlossen werden, von denen zumindest eine ein Ventil aufweist, sodass die das Element (1) enthaltende Hülle nach Anpassung an den Verletzten evakuierbar ist.

## Claims

1. Method for filling a flexible element (1) with a loose granular material (9), wherein the element (1) has two lengths of material (2) connected to each other at points (3) in the interior (4) and along the edge, leaving a filling opening (5), **characterized in that** an air pressure difference between the interior (4) and the external environment is generated, which widens the interior (4) before it is filled with the granular material (9).

2. Method according to claim 1, **characterized in that** the air pressure difference between the interior (4) and the external environment is produced by suctioning off ambient air.

3. Method according to claim 2, **characterized in that** the element (1) is introduced into a vacuum chamber (10), wherein the filling opening (5) is accessible from the outside, and the vacuum, which widens the interior (4), is generated in the vacuum chamber (10).

4. Method according to claim 1, **characterized in that** the air pressure difference between the interior (4) and the external environment is produced by blowing air in through the filling opening (5).

5. Method according to claim 4, **characterized in that** the loose granular material (9) is added to the stream of air introduced through the filling opening (5).

6. Method according to claim 2 or 4, **characterized in that** the loose granular material (9) is introduced through the filling opening (5) by means of gravity after the widening of the interior (4).

7. Method according to one of claims 1 to 6, **characterized in that** the filling opening (5) is provided in a fill nozzle (6) formed of areas of the two lengths of material (2), which is closed after the granular material (9) has been introduced.

8. Method according to claim 3 and 7, **characterized in that** the element (1) is suspended from the fill nozzle (6) in the vacuum chamber (10).

9. Method according to one of claims 1 to 8, **characterized in that** the granular material (9) consists of foamed polystyrene with a weight of between 20 kg/m³ and 70 kg/m³.

10. Method according to one of claims 1 to 8, **characterized in that** the granular material (9) consists of foamed polystyrene with a diameter of between 0.4 mm and 5 mm.

11. Method according to claim 9 or 10, **characterized in that** the granular material (9) consists of foamed polystyrene with a weight of 60 kg/m³ and a diameter of between 0.5 mm and 2 mm.

12. Method for producing a flexible element (1) for supporting and stabilizing an injured person, in which a method according to one of claims 1 to 11 for filling the flexible element (1) is used.

13. Method according to claim 12, **characterized in that** the two lengths of material (2) are formed of air-tight films, at least one of which has a valve, with the result that the element (1) filled with the granular material (9) can be evacuated after it has been fitted to the injured person.

14. Method according to claim 12, **characterized in that** the two lengths of material (2) are formed of an air-permeable non-woven, and after filling with the granular material (9) are enclosed in a shell consisting of air-tight films, at least one of which has a valve, with the result that the shell containing the element (1) can be evacuated after it has been fitted to the injured person.

## Revendications

1. Procédé de remplissage d'un élément (1) souple pour supporter et stabiliser une personne blessée avec un matériau granulaire (9) en vrac, dans lequel l'élément (1) présente deux bandes de matériau (2) reliées l'une à l'autre - en des emplacements (3) dans l'espace intérieur (4) et, en laissant une ouverture de remplissage (5), le long du bord - **caractérisé en ce qu'**une différence de pression d'air entre l'espace intérieur (4) et l'environnement extérieur est générée, laquelle élargit l'espace intérieur (4) avant d'être rempli avec le matériau granulaire (9).

2. Procédé selon la revendication 1, **caractérisé en ce que** la différence de pression d'air entre l'espace intérieur (4) et l'environnement extérieur est établie par évacuation par aspiration de l'air ambiant.

3. Procédé selon la revendication 2, **caractérisé en ce que** l'élément (1) est inséré dans une chambre de dépression (10), dans lequel l'ouverture de remplissage (5) est accessible depuis l'extérieur, et la dépression, qui élargit l'espace intérieur (4), est générée dans la chambre de dépression (10).

4. Procédé selon la revendication 1, **caractérisé en ce que** la différence de pression d'air entre l'espace intérieur (4) et l'environnement extérieur est établie par soufflage d'air par l'ouverture de remplissage (5).

5. Procédé selon la revendication 4, **caractérisé en ce que** le matériau granulaire (9) en vrac est ajouté au flux d'air introduit par l'ouverture de remplissage (5).

6. Procédé selon la revendication 2 ou 4, **caractérisé en ce que** le matériau granulaire (9) en vrac est introduit au moyen de la gravité par l'ouverture de remplissage (5) après l'élargissement de l'espace intérieur (4).

7. Procédé selon l'une quelconque des revendications 1 à 6, **caractérisé en ce que** l'ouverture de remplissage (5) est prévue dans une tubulure de remplissage (6) formée à partir de zone des deux bandes de matériau (2), laquelle est fermée après l'introduction du matériau granulaire (9).

8. Procédé selon la revendication 3 et 7, **caractérisé en ce que** l'élément (1) est suspendu sur la tubulure de remplissage (6) dans la chambre de dépression (10).

9. Procédé selon l'une quelconque des revendications 1 à 8, **caractérisé en ce que** le matériau granulaire (9) est constitué d'une mousse de polystyrène avec un poids entre
20 kg/m³ et 70 kg/m³.

10. Procédé selon l'une quelconque des revendications 1 à 8, **caractérisé en ce que** le matériau granulaire (9) est constitué d'une mousse de polystyrène avec un diamètre entre 0,4 mm et 5 mm.

11. Procédé selon la revendication 9 ou 10, **caractérisé en ce que** le matériau granulaire (9) est constitué d'une mousse de polystyrène avec un poids de 60 kg/m³ et un diamètre entre 0,5 mm et 2 mm.

12. Procédé de fabrication d'un élément (1) souple pour supporter et stabiliser une personne blessée, où un procédé selon l'une quelconque des revendications 1 à 11 est appliqué pour remplir l'élément (1) souple.

13. Procédé selon la revendication 12, **caractérisé en ce que** les deux bandes de matériau (2) sont formées de films étanches à l'air, dont au moins un présente une vanne, si bien que le vide peut être fait dans l'élément (1) rempli du matériau granulaire (9) après adaptation à la personne blessée.

14. Procédé selon la revendication 12, **caractérisé en ce que** les deux bandes de matériau (2) sont formées à partir de non-tissé laissant passer l'air et sont enfermées dans une enveloppe composée de films étanches à l'air après le remplissage avec le matériau granulaire (9), dont au moins un présente une vanne si bien que le vide peut être réalisé dans l'enveloppe contenant l'élément (1) après adaptation à la personne blessée.
